# EUROPEAN PATENT APPLICATION

(11) **EP 4 607 174 A1**
(43) Date of publication of application: **27.08.2025**
(21) Application number: 24162158.0
(22) Date of filing: 07.03.2024
(51) Int. Cl.: G01N 1/40, A61B 10/00, B01D 29/11, B01L 3/00

(54) **SAMPLE PROCESSING DEVICE AND METHOD OF USE THEREOF**

(30) Priority: 22.02.2024 GB 202402539
(71) Applicant: Reardon, Robert John Fraser, London SW7 4SP (GB)
(72) Inventor: Reardon, Robert John Fraser, London SW7 4SP (GB)
(74) Representative: Schlich

(57) **Abstract**

A device for collecting buoyant material is described wherein the device comprises a body having a first end and a second end and walls therebetween defining an internal space, wherein the first end has a first portal therein, wherein the second end has a second portal therein, wherein the port in the first end is in fluid connection with the space, wherein the port in the second end is in fluid connection with the space, wherein the fluid connection between the first and second portals is occluded by a filter or mesh mounted at the second end or between the first and second ends to extend over a transverse area of the space at the second end or between the first and second ends, wherein the cross sectional area of the space narrows between the second end and the first end, and wherein the exterior dimensions of the of the second end of the device are for slidable installation or seating in a cooperating vessel. Also described is a method of using the device for concentrating buoyant material.

## Description

The present invention relates to equipment for processing and extracting particular elements from materials. In particular, the devices and apparatuses of the present invention are suitable for processing semisolid materials and in particular biological samples, most particularly faecal or stool samples. The samples may be processed by releasing buoyant material from the sample into a liquid medium and then concentrating the buoyant material by constraining the area of the exposed liquid that the buoyant material must ascend into.

Accordingly, the present invention also relates to methods of processing and sampling materials obtained using the devices and apparatus of the present invention. Accordingly, the invention also comprises methods for subsequently processing such samples, in particular biological samples, most particularly faecal or stool samples. These methods are particularly for use in the context of laboratory use. Thus, the cooperating vessels are suitably laboratory tubes.

Testing of faecal matter is a common diagnostic procedure used to detect internal parasites, especially in veterinary species. This is because of the ease of obtaining sample material and the tests are relatively low-cost and relatively straightforward to conduct.

The majority of parasite ova/ eggs have a specific gravity (SG) that falls between 1.05 and 1.23, thus the faecal flotation relies on the ascension of eggs and ova using the difference in overall density to the flotation solution. The most common flotation solutions used in veterinary medicine include saturated sodium chloride, sodium nitrate, sugar (e.g. Sheather's solution), magnesium sulphate, and zinc sulphate.

Sodium nitrate typically has an SG of 1.2 - 1.33. Zinc sulphate (ZnSO4) typically has an SG of 1.18. Saturated sodium chloride (N HCl) typically has an SG 1.2. Saturated Epsom salt (magnesium sulphate; MgSO3). Sheather's solution typically has an SG of 1.25.

Existing methods require pre-processing of faecal matter before suspensions of matter can be prepared and then allowed to stand, rest, or incubate so that buoyant parasites or parasite eggs (oocytes) can be collected from the surface of the liquid. Alternatively or in in addition the separation of buoyant and non-buoyant materials can be enhanced by the centrifugation of the sample-containing suspension. For example, methods utilising centrifugation aid in the separation of helminth eggs and protozoan oocysts in a solution versus passive relying completely on the density and specific gravity for ascension of the materials of interest (e.g. helminth eggs and protozoan oocysts) to the surface of the liquid.

Thus there exists a need for methods of processing biological materials, and in particular faecal or stool samples that yield extracts from the material being tested that are more amenable to testing or yield improved test results in terms or accuracy and/or reduction of variability. In this way it is also an object of the invention that the improved test material might be further and/or better integrated with subsequent testing processes.

Accordingly, there is also a need for devices that enable such methods and/or can be used in concert with such methods to achieve these objectives.

In addition, there is a need for reduced variability between testing procedures. Hereto now variability in testing of such biological materials has been less of a problem because data produced within a laboratory would be expected to be internally consistent. Furthermore, many tests depend on other techniques such as microscopy and thus there are independent methods of ascertaining a particular result or confirming an unexpected or difficult result.

However, there is a move towards large reference laboratories who will take in samples from a wide variety of places and must produce results that can be comparable and standardised. Furthermore, such results are suitable for automated testing and diagnosis which offers the prospect of more rapid and reliable testing, as well as being less costly.

The invention provides a device for collecting buoyant material comprising a body having a first end and a second end and walls therebetween defining an internal space,
wherein the first end has a first portal therein,
wherein the second end has a second portal therein,
wherein the port in the first end is in fluid connection with the space,
wherein the port in the second end is in fluid connection with the space,
wherein the fluid connection between the first and second portals is occluded by a filter or mesh mounted at the second end or between the first and second ends to extend over a transverse area of the space at the second end or between the first and second ends,
wherein the cross sectional area of the space narrows between the second end and the first end, and
wherein the exterior dimensions of the of the second end of the device are for slidable installation or seating in a cooperating vessel.

The side wall or walls of the device may define the edge of the first and/or second portal. Thus the first portal may be substantially a hollow and open-ended pipe or varying cross-sectional area. Thus the second portal may be substantially a hollow and open-ended cylinder.

Accordingly, the body may be substantially hollow.

The second end may be wider than the first end. The cross sectional area of the portal at the first end may be smaller than the cross sectional area of the portal at the second end.

The body may have internal and external side walls. The body may have a side wall whose exterior surface defines the part or substantially all of the exterior of the device and whose interior surface defines the part or substantially all of the internal space of the device.

The device may be installed in the cooperating vessel with a plug fit. Thus the second end of the device has suitable outer dimensions such that it can be inserted in the cooperating vessel and the surface or surfaces of the device body that abut the inner walls of the device match the contours of the inner walls of the cooperating vessel. Preferably, the device has a plug fit at a predetermined position in the cooperating vessel, which is most preferably a laboratory tube.

The circumference of the second end may seal against the inside walls of the cooperating vessel. Advantageously this improves the ability of the device to generate or maintain pressure changes in the internal space of the device when the device urged towards the base of the cooperating vessel.

The inner wall or walls of the portal of the second end may be perpendicular to the longitudinal axis of the body. This form has the advantage of not simpler removal from an injection moulding tool and providing a defined bore for buoyant material to ascend through.

The passage through the device from the upper surface to the lower surface may be straight. Preferably, the passage is parallel with the central axis of rotation of the cooperating laboratory tube. Most preferably, the passage is co-linear with the central axis of rotation of the cooperating laboratory tube but may be of varying cross-sectional area. This form has the advantage of not simpler removal from an injection moulding tool and providing a defined bore for buoyant material to ascend through.

The device may be for insertion in a laboratory tube. Thus the device of the present invention may be suitable for cooperative use with a compatible laboratory tube. That is, the device has suitable outer dimensions such that it can be inserted in a laboratory tube and the surface or surfaces of the device that abuts the inner walls of the laboratory tube match the contours of the laboratory tube. Preferably, the device has a plug fit at a predetermined position in the laboratory tube. In this way, the position in which the device is seated in the laboratory tube can be altered in order that a specific volume can be defined in the space defined and/or substantially bounded by the lower surface of the device and the walls of the laboratory tube.

Thus the invention provides an apparatus wherein the device described herein is installed in a compatible vessel, preferably a laboratory tube.

Laboratory tubes are conveniently used for holding and processing volumes of liquids. These may be samples of biological origin or other liquids. Accordingly, the definition of liquid in the context of the present disclosure includes pure liquids and also suspensions of matter in liquids. Accordingly, the device disclosed herein may be suitable for operation in the context of liquids and/or suspensions.

The vessel may be of the standard volumes and/or dimensions of laboratory tubes produced by Eppendorf^{®}, Stirling^{®}, Falcon^{®}; or generic versions and/or variants thereof. Thus, advantageously, the device may be made to be compatible with a wide range of standard and widely used laboratory tubes. Accordingly the apparatus described herein may advantageously be compatible with existing and/or standard laboratory equipment, e.g. centrifuges.

Accordingly, the laboratory tube may be round, elliptical, or circular in transverse cross-section. Preferably, the laboratory tube is substantially circular in transverse cross-section.

Thus the laboratory tube may be designed to have a total volume of 50 ml, 15 ml, 10 ml, 2.5 ml, 2 ml, 1.5 ml, 1 ml, or 0.5 ml.

In the context of the present disclosure, the term "for" is defined to mean "suitable for" the purpose, method or objective defined in conjunction with this term.

In the context of the present disclosure, the term "slidably" means capable of sliding or of being slid. In the case of the present invention this may be in particular being moved axially with respect to the device.

The cross sectional area of the space may narrow linearly for all or a portion of the distance between the second end and the first end.

The cross sectional area of the space may narrow to a cylindrical pipe.

The cross sectional area of the space may narrow discontinuously between the second end and the first end.

Thus the contours or profile of the walls of the space allow for narrowing of the cross sectional area of the space in a variety of forms. Thus a form that is advantageous for isolation and concentration of a particular material may be utilised. Without wishing to be bound by theory, the form of the walls of the space may be formed to speed the ascent of materials intended for testing or measurement while restricting the ascent of material that are not desirable or might contaminate the further testing or measurement steps.

Thus the first and second portals are in fluid connection and the fluid connection between the first and second portal may be partially obstructed by the filter or mesh material

The filter or mesh may be mounted to extend over substantially the whole of a transverse area of the space between the first and second ends. Thus, advantageously the filter has effect over the whole of the cross-sectional area of the device and substantially no areas are blocked from contributing material to the final filtrate (filtered product).

Filter or mesh may be mounted substantially orthogonal to the longitudinal axis of the device. Thus, advantageously ascending buoyant material is not made to deviate towards the walls of the device. The filter or mesh may be mounted at the mid-point on the longitudinal axis of the body. Thus the body may advantageously be symmetrical and the first and second ends interchangeable leading to simplified use by the user.

The filter or mesh may be planar. Thus the body may advantageously be symmetrical and the first and second ends interchangeable leading to simplified use by the user.

The mesh may be a grid with an array of apertures arranged in a square array ("grid") or other regularly spaced array (e.g. hexagonal). The apertures in the array may be square, rounded, or circular. The apertures may have widths of 150µm to 600µm. Square apertures in the array may have sides of 150µm to 600µm. Circular apertures in the grid may have diameters of 150µm to 600µm.

Square apertures in the array may have sides of lengths selected from 150µm 175 µm, 200 µm, 225 µm, 250 µm, 275 µm, 300 µm, 325 µm, 350 µm, 375 µm, 400 µm, 425 µm, 450 µm, 475 µm, 500 µm, 525 µm, 550 µm, 575 µm, or 600µm. Circular apertures in the array may have of diameters selected from 150µm 175 µm, 200 µm, 225 µm, 250 µm, 275 µm, 300 µm, 325 µm, 350 µm, 375 µm, 400 µm, 425 µm, 450 µm, 475 µm, 500 µm, 525 µm, 550 µm, 575 µm, or 600µm. Square apertures in the array may have sides of 600 µm. Square apertures in the array may have sides of 250 µm

The material of the array may be composed of intervening or supporting members of widths selected from 20 µm, 40 µm, 60 µm, 80 µm, 100 µm, 120 µm, 125 µm, 130 µm, 140 µm, 150 µm, 160 µm, 170 µm, 175 µm, 180 µm, 200 µm, 220 µm, 225 µm, 230 µm, 240 µm, 250 µm, 260 µm, 270 µm, 275 µm, 280 µm, 300 µm, 320 µm, 325 µm, 330 µm, 340 µm, 350 µm, 360 µm, 370 µm, 375 µm, 380 µm, 400 µm, 420 µm, 425 µm, 430 µm, 440 µm, 450 µm, 460 µm, 470 µm, 475 µm, 480 µm, or 500 µm.

The material of the mesh or filter may be composed of intervening or supporting members of 200 µm width. The material of the array may be composed of intervening or supporting members of 200 µm width

The proportion of open area of the mesh or filter may be in the range 30% - 40%, 40% - 50%, 50% - 60%, 60% - 70%, 70% - 80%, or 80% - 90%. The proportion of open area of the mesh or filter may be selected from 20%, 25%, 30%, 35%, 40%, 45%, 50%, 55%, 60%, 65%, 70%, 75%, or 80% open area.

The mesh or filter may have square apertures with sides of 250 µm and intervening or supporting members of 100 µm width.

The mesh or filter may have square apertures with sides of 600 µm and intervening or supporting members of 200 µm width.

Thus advantageously the device may be configured or manufactured to have a wide range of filtering capacities and/or be configured or manufactured to have specific filtering characteristics. Thus advantageously the characteristics of filtering capacity and/or filtering speed may be optimised for particular sample substances and filtering processes.

The filter or mesh may be ridged or contoured. Thus the filter or mesh may be three-dimensional in form. Advantageously, a three dimensional form yields greater surface area for filtration. An additional advantage is that small slopes in a three dimensional profile allows release of smaller entities to float upwards rather than being trapped beneath a planar part of the filtering mesh.

The apparatus may have a lid or cap portion which may be installed in or on the first end of the device. The cap may also, advantageously, be used to seal the device and the cooperating vessel that the device is installed in. Thus the body of the cap may be surmounted by a lid section (or flange) of greater radius than the body of the cap. The lid section may have a rim projecting perpendicularly from the circular lid section in the direction of the body of the cap.

In the present context the term space encompasses the terms void or empty volume.

In the present context the term fluid connection is defined as a path for the flow of fluid, which may be a gas or liquid, preferably a liquid.

In the present context the term portal means a hole, tunnel, or passage. Preferably the portal, hole or tunnel has walls aligned with or parallel with the longitudinal axis of the body. Most preferably the portal is circular or elliptical.

In the present context the term transverse means acting, lying, or being across, set crosswise, preferably the transverse plane is made at right angles to the long axis of the body

In the present context the term a sample means an amount of material, advantageously, this may be a pre-determined amount of material.

In the present context the term occluded means a flow path or fluid connection is partially obstructed by an impediment, for example a filter or mesh.

In the present context the terms filter or mesh may also encompass the terms strainer or grid.

The device may be made of plastics material. Preferably, the plastics material is injection moulded. however, the plastics material may be 3-D printed. The device may be manufactured from one or more plastics material selected from the following list: acrylic (PMMA); acrylonitrile butadiene styrene (ABS)
polyamide (PA; nylon); polycarbonate (PC); polyethylene (PE); polyoxymethylene (POM); polypropylene (PP); polystyrene (PS); thermoplastic elastomer (TPE); or thermoplastic polyurethane (TPU). Preferably the plastics material selected is polypropylene. Preferably the plastics material selected is polyethylene, most preferably the plastic material is high density polyethylene (HDPE).

The plastics material may be biologically inert. In this context biologically inert means that the plastics material does not react with biological material that it comes into contact with. Accordingly, such a biologically inert material does not affect the results of tests, or assays carried out on biological samples or liquids that it is brought into contact with. More preferably these are plastics materials which are characterised by low absorbance of protein, such as polypropylene and/or polycarbonate.

The invention also provides a method for concentrating buoyant material, comprising the steps of:
a) immersing a sample of material in a liquid held within a cooperating vessel;
b) installing a device for concentrating buoyant biological material in the cooperating vessel, wherein the device comprises a body having a first end and a second end and walls therebetween defining an internal space,
   wherein the first end has a first portal therein;
   wherein the second end has a second portal therein;
   wherein the port in the first end is in fluid connection with the space;
   wherein the port in the second end is in fluid connection with the space;
   wherein the fluid connection between the first and second portals is occluded by a filter or mesh mounted at the second end or between the first and second ends to extend over a transverse area of the space at the second end or between the first and second ends
   wherein the cross sectional area of the space narrows between the second end and the first end
   wherein the exterior dimensions of the of the second end of the device are for slidable installation or seating in a cooperating vessel; and
c) wherein the second end of the device is installed first in the cooperating vessel
d) urging the device towards the base of the cooperating vessel such that the filter or mesh is submerged in the liquid and the meniscus of the liquid is driven upwards into the region of the device between the second end and the first end where the cross sectional area of the space narrows.

Thus the method has the advantage of concentrates the material floating at the air-liquid interface (i.e. at the meniscus) when the material is carried with the liquid driven into the region of the device where the cross sectional area of the space narrows reduces the area of the meniscus.

The method may comprise the step of sampling 1 to 5 grams (g) of material. The method may comprise the step of sampling 1 to 3 grams (g) of material. The method may comprise the step of sampling an amount of material of 1 g, 2 g, 3 g, 4 g, or 5 g.

In this context a vessel is a container that is impermeable to the passage of liquid through the walls thereof.

The liquid may be a reagent for preservation or testing of materials.

The reagent or liquid may have a specific gravity of 1.18 to 1.26. The reagent may have a specific gravity of 1.17, 1.18, 1.19, 1.20, 1.21, 1.22, 1.23, 1.24, 1.25, 1.26, 1.37, 1.28, 1.29, 1.30, 1.31, 1.32, 1.33, or 1.34.

The method may additionally comprise the step of mixing or homogenising the material with the liquid. The method additionally comprising the step of agitating or mixing the sample in the liquid

The method may additionally comprise the step of incubating the material in the liquid. This allows time for buoyant material to rise to the surface of the liquid. This may be via the perforations or apertures in the mesh or filter of the device. Advantageously, larger yet buoyant material is retained underneath the mesh or filter so that the material recovered from the surface of the liquid is less contaminated.

The method may additionally comprise the step of capping the device. The cap may be removed before the material is removed from the first end

The method may additionally comprise centrifuging the vessel.

The method may comprise the step of allowing the apparatus to stand so that, advantageously, the buoyant material being filtered can ascend by flotation to be concentrated at the surface of the liquid (e.g. the meniscus).

The method may additionally comprise the step of collecting a sample of liquid or material from the upper surface of the liquid (e.g. the meniscus).

The biological material being tested for may be selected from or include helminths and/or eggs thereof, protozoa, protozoa and eggs/cysts, giardia, lung worm larvae, Cryptosporidium, tapeworm eggs, nematode eggs. The buoyancy of these materials may be achieved or optimized by immersing the sample material in a liquid of a defined specific gravity in which the desired biological material has positive buoyancy.

Once the buoyant material has migrated from the liquid to the upper surface thereof, then all or part of that material may be removed, typically being carried within a volume of liquid or suspension. Removal of the liquid can be achieved by a number of standard means. Preferably, this is by means of insertion of a pipette tip, tube, or conduit in the liquid, typically at the upper surface and the generation of a partial vacuum in the pipette tip by the operation of a piston or suction developed by the reinflation of a pipette bulb. Accordingly, the pipe may be a pipette tip or hollow (e.g. hypodermic) needle. Alternatively or in addition the buoyant material may be removed from the mass of liquid by the use of an inoculation loop and the surface tension of the liquid

The method may additionally comprise a step of carrying out a further test or tests on the sample of liquid or material from the upper surface of the liquid. These tests may be done using microscopy. This may be light microscopy. Thus advantageously the processes sample may be analysed using a further test or tests to discern the presence and/or prevalence and/or frequency of particular materials in the sample. Preferably these materials are biological materials. The additional testing yield information regarding the identity and/or frequency of particular biological materials in the sample. Most preferably the biological material is selected from helminths and/or eggs thereof, protozoa, protozoa and eggs/cysts, giardia, lung worm larvae, Cryptosporidium, tapeworm eggs, nematode eggs. The additional test may be a staining test to increase the visibility and or detection of particular biological materials.

Alternatively or in addition the samples may be analysed using an artificial intelligence trained to discern the presence and/or prevalence and/or frequency of particular materials in the sample. Preferably these materials are biological materials Most preferably the additional testing yield information regarding the identity and/or frequency of particular biological materials in the sample. The additional testing yield information regarding the identity and/or frequency of particular biological materials in the sample. Most preferably the biological material is selected from helminths and/or eggs thereof, protozoa, protozoa and eggs/cysts, giardia, lung worm larvae, Cryptosporidium, tapeworm eggs, nematode eggs. Staining or equivalent procedures may be used to increase the visibility and or detection of particular biological materials.

The terms seats and seated are defined in the present context to mean to be fitted in position. Further, in the context of the present disclosure that means that a device is fitted in the correct and/or predetermined position in the cooperating laboratory tube.

Seating of the device in the cooperating laboratory tube may be achieved via axial pressure on the device towards the base of the cooperating laboratory tube. The force required for this seating may be provided by centrifugation of the laboratory tube containing the device.

The material to be sampled may be solid or semi solid. The material to be sampled may be a liquid or a suspension. Advantageously a wide variety of samples may be filtered or otherwise processed according to the present invention.

The material to be sampled is solid or semi solid that is the material may be quasi-solid, falsely-solid, or semisolid being the physical term for something whose state lies between a solid and a liquid. Thus a semi solid material is similar to solids in some respects, such as having the ability to support its own weight and hold its shape, a quasi-solid also shares some properties of liquids, such as conforming in shape to something applying pressure to it and the ability to flow under pressure, be that positive or negative pressure. The words quasi-solid, semisolid, and semiliquid may be used interchangeably. Quasi-solids and semisolids are sometimes described as amorphous because at the microscopic scale they may have a disordered structure, in contrast to crystalline solids. They should not be confused with amorphous solids as they are not solids and exhibit properties such as flow which bulk solids do not.

The substance processed using the device, apparatus and/or methods described herein may be a biological sample. Thus, the apparatus and methods disclosed herein are suitable for processing of biological samples, e.g. stool, faeces (feces), human or animal tissue, urine, blood, lymph, plasma, cerebrospinal fluid, and saliva. Preferably, the sample is a stool or faecal sample. The substance or sample may be mixed with a solution or reagent that may be selected from a diluent, a buffer, a fixative, or a test reagent. The present device, apparatus and/or methods described herein are particularly well suited to processing and analysis of stool or faecal samples.

Additional advantages of the methods described herein, and devices and apparatus therefor, are increased sensitivity when processing samples containing a rare protozoan. Similarly the methods described herein, and devices and apparatus therefor, yield increased recovery of "scanty ova" (i.e. rarely occurring (scant) ova of pathogens, e.g. protozoa, in the sample) from sample materials. These improvements also yield easier morphological examination of pathogens or other materials in the processed material.

Further advantages of using the methods described herein, and devices and apparatus therefor, are increased efficiency of laboratory operations in terms of throughput, and in terms of achieving improved on-demand and patient-driven workflows.

In this context the term sieving refers to a process for separating wanted elements from unwanted material or for controlling the particle size distribution of a sample, by using a screen such as a mesh, net, or perforated material. Thus sieving includes the act of passing matter through a sieve to change the particle size distribution, viscosity, or consistency of a sample. Thus sieving may be used to remove parts of a sample that are larger than the apertures in the sieve. Sieving also includes collecting the material, liquid and/or solid, from such a process that has passed through the sieve.

In this context, a filter is a porous device for removing solid particles from a liquid passed through it. Thus a sieve is a form of coarse filter. Thus a filter may comprise a filter medium which is preferably a porous article or membrane through which liquid may be passed in order to separate out matter therefrom. Thus filtering includes the act of material passing through a filter to remove other material therefrom. Thus, filtering also includes collecting the liquid filtrate from such a process including the material carried in the filtrate.

Accordingly, the process of filtering material also includes the process of retaining material behind or beneath a filter or mesh that is partially or completely immersed in a liquid while buoyant material ascends through the mesh or fileter. In this way buoyant and non-buoyant materials can be separated. Also, in this way buoyant materials smaller than the apertures in the mesh or filter can be separated from larger particles or pieces of buoyant material.

The invention also provides a kit comprising a device and cooperating vessel, such as a laboratory tube, for combining into an apparatus as described herein.

The device described herein may be manufactured by techniques such as injection moulding of plastics material to produce the devices in large numbers. This simplicity of manufacture has the attendant advantage that the device may be disposable. Similarly the cooperating vessels may be manufactured by techniques such as injection moulding of plastics material.

The invention provides an injection moulding tool for manufacturing a device as described herein.

Injection moulding of devices of the invention is advantageous because it yields products with monolithic, consistent internal structure that minimises the occurrence of flaws or weak spots in the products that might occur in multi-part devices. Such flaws or weak spots might be exposed under centrifugation or differential pressure and result in failure of the device.

The methods of manufacture of devices of the invention or components thereof may comprise the step of applying filter or semipermeable material to pre-determined portions of the injection moulding tool, closing the tool, admitting plastics material to the void formed by closing the tool, allowing the plastics material to solidify, and removing the product from the tool wherein the filter or semipermeable membrane is incorporated and/or secured in the plastics material of the product in a pre-determined position.

This process yields the advantage of injection moulding of forming the device and incorporating the filter or semipermeable membrane into the device in one step. This is known as over moulding (overmoulding).

### Examples and Drawings

The invention will now be described with reference to the following drawings and examples in which:

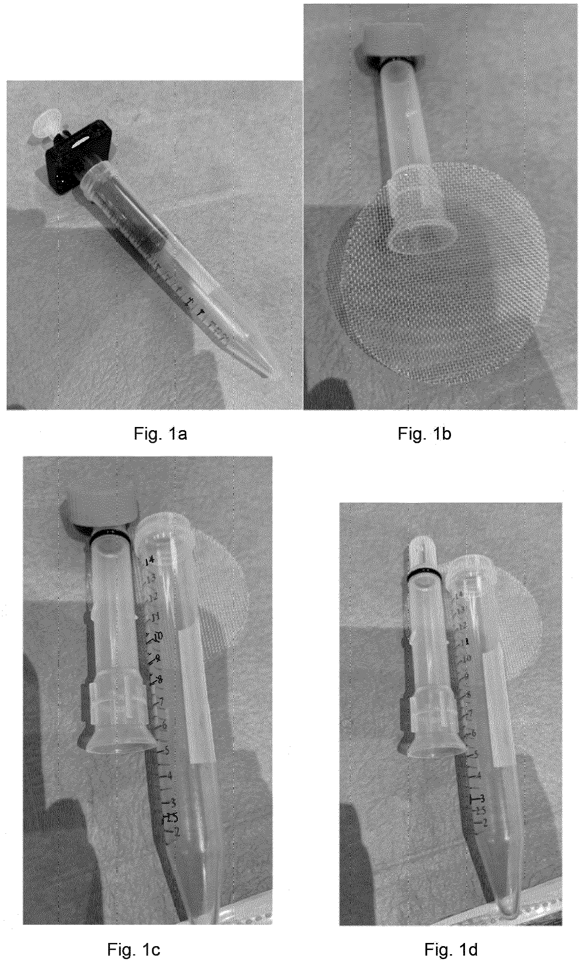

- Fig. 1: shows photographs documenting the development of the device. Fig. 1a shows a laboratory tube and sample injector inserted therein. Fig. 1b shows an example of a device as described herein surmounted by a cap with a section of mesh or filter material separate therefrom. Fig. 1c shows an example of a device as described herein surmounted by a cap next to a cooperating laboratory tube. Fig. 1c shows an example of an uncapped device as described herein next to a cooperating laboratory tube.

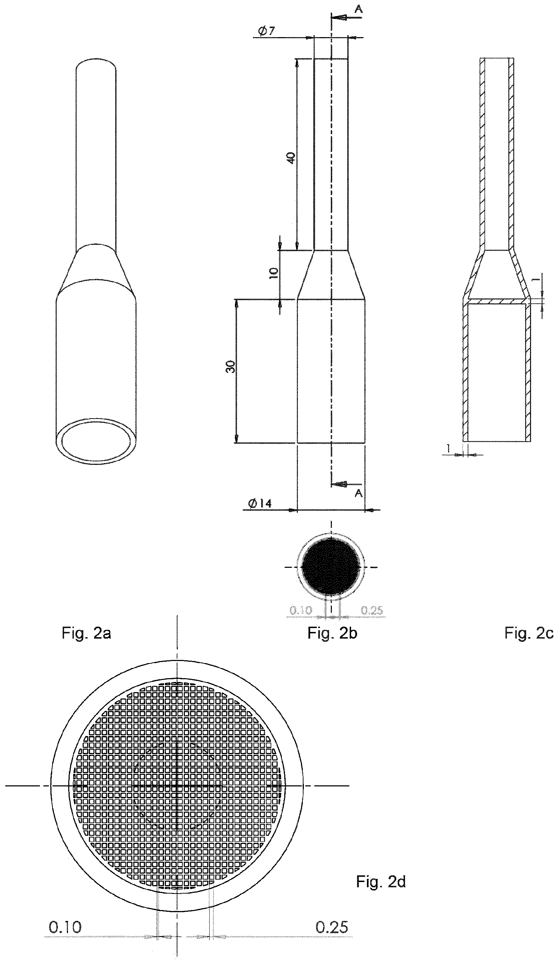

- Fig. 2: shows diagrams of different views of the device as described herein. Fig. 2a shows an isometric view of the exterior of the device. Fig. 2b shows a side view of the device of Fig. 2a. Fig. 2c shows a cross-section along line A-A of Fig. 2b. Fig. 2d shows a longitudinal view of the device from the wider end of the device and shows the transverse mesh separating the upper and lower chambers of the device shown in Fig. 2c

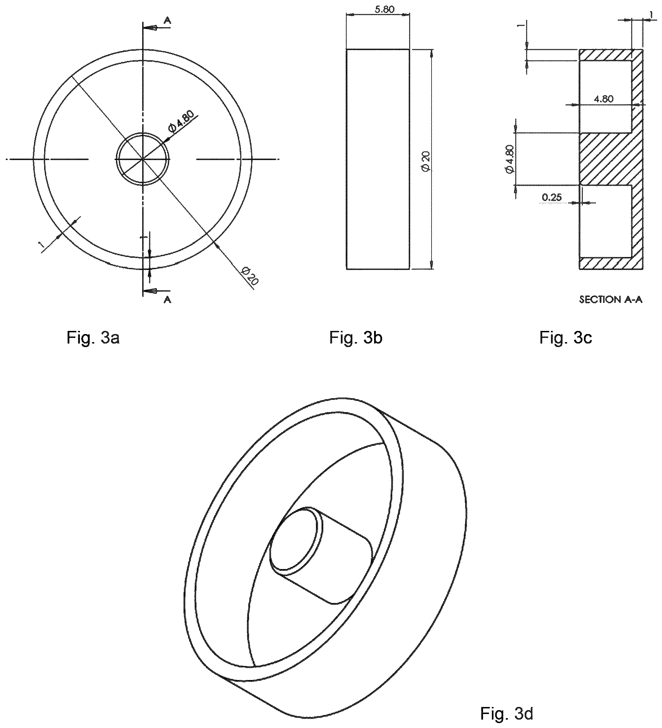

- Fig. 3: shows diagrams of views of a cap that cooperates with the device of Fig. 3. Fig. 3a shows a top view of the cap. Fig. 3b shows a side view of the cap. Fig. 3c shows a cross-section along line A-A of Fig. 3a. Fig. 3d shows an isometric view of the cap.

### Example 1

The device shown in Figure 2 is comprised of two substantially cylindrical sections of different radii linked by a hollow conic or funnel-like section. The device may be made of polyethylene or polypropylene. In the present example the sidewalls of the device are 1 mm thick, the device has an exterior diameter of 14 mm and an overall length of 80 mm. the other, narrower cylindrical section is 40 mm in length. The lower, broader cylindrical section is 30 mm in length. The linking hollow conical section is 10 mm in length/height. In a transverse plane at the broader base of the hollow conic section a mesh is mounted transversely across the whole transverse area of the cylinder. The mesh comprises an array of substantially square apertures of side length 0.25 mm separated by members of 0.1 mm width.

Typically a faeces or stool sample will be obtained in a clinical or veterinary setting, e.g. a clinic or hospital. Preferably the cooperating vessel contains a liquid test medium or preservation medium. Thus 1 to 5 grams of sample can be obtained, measured by weight or volume if desired, and transferred to a cooperating vessel. Preferably this is a 15 ml Falcon laboratory tube.

The sample is then mixed and/or emulsified in the liquid by shaking or vortexing.

Optionally, the assembly of laboratory tube, device, sample, and cap is centrifuged to sediment the heavier, non-buoyant materials in the sample. Typically the assembly of laboratory tube, device, sample, and cap is centrifuged to sediment the heavier, non-buoyant materials in the sample.

The device is installed by sliding the second, broader end of the device into the cooperating laboratory tube and urging or pushing the device towards the base of the cooperating vessel such that the filter or mesh is submerged in the liquid and the meniscus of the liquid is driven upwards into the region of the device between the second end and the first end where the cross sectional area of the space narrows.

Conveniently the device may be translucent or transparent. Accordingly, the position of liquid and/or liquid level within the device may be observed or measured. Thes observations or measurements may be assisted by the use of measurements or graduations marked or moulded along the side of the device (not shown).

The assembly is than allowed to stand or incubate to allow buoyant material to ascend through the liquid.

Accordingly, buoyant material ascending from the sample contained in the lower chamber via the mesh or filter enters a volume in which the area of the meniscus is reduced and thus the concentration of buoyant material found therein is increased. This increasing concentration has necessary advantages for simplification of analysis of the biological material obtained thereby.

The buoyant material (which might include helminths and/or eggs thereof, protozoa, protozoa and eggs/cysts, giardia, lung worm larvae, Cryptosporidium, tapeworm eggs, nematode eggs) is then allowed to ascend to the surface of the liquid in the tube by allowing the tube to stand for the time necessary for this to occur.

The buoyant material may then be extracted for further testing.

If the buoyant material is to be tested by light microscopy then the level of the meniscus is brought level with the portal of the first end. A glass slide or coverslip is then placed on top of the tube in contact with the liquid and allowed to stand for at least 20 minutes. The slide or cover slip is then lifted off and inverted for examination by light microscopy.

Typically the light microscopy would be conducted by covering the drop retain from the meniscus on the slide or coverslip with a cooperating coverslip or slide and then examining the sample under a light microscope at 10× and/or 40× magnification, with the condenser in the down position, under low light.

Alternatively, if the buoyant material is to be tested by automated methods then the cap is removed and a sample of the liquid from the meniscus may be obtained using an inoculation loop or a pipette such that the liquid obtained can be transferred to the test chamber or surface of the test equipment. Typically, automated methods entail the use of image recognition by an artificial intelligence trained to discern and/or recognise the identity and/or frequency of particular organisms, eggs, or other biological entities existing in the sample.

The faecal or stool sample may then be then stored in a fixative agent, such as formaldehyde for storage and/or later analysis.

Thus the invention provides improved methods of processing biological materials, and in particular faecal or stool samples. The invention further provides devices and apparatus for use in concert with these methods of the invention.

## Claims

1. A device for collecting buoyant material comprising a body having a first end and a second end and walls therebetween defining an internal space,
wherein the first end has a first portal therein,
wherein the second end has a second portal therein,
wherein the port in the first end is in fluid connection with the space,
wherein the port in the second end is in fluid connection with the space,
wherein the fluid connection between the first and second portals is occluded by a filter or mesh mounted at the second end or between the first and second ends to extend over a transverse area of the space at the second end or between the first and second ends, s
wherein the cross sectional area of the space narrows between the second end and the first end, and
wherein the exterior dimensions of the of the second end of the device are for slidable installation or seating in a cooperating vessel.

2. A device according to claim 1, wherein the circumference of the second end seals against the inside walls of the cooperating vessel.

3. A device according to claim 1 or claim 2, wherein the filter or mesh comprises a regularly spaced array of apertures.

4. A device according to any preceding claim, wherein the apertures have widths of 150µm to 600µm.

5. A device according to any preceding claim, wherein the cross sectional area of the space narrows linearly for all or a portion of the distance between the second end and the first end.

6. A device according to any preceding claim, wherein the cross sectional area of the space narrows to a cylindrical pipe.

7. A device according to any preceding claim, wherein the cross sectional area of the space narrows discontinuously between the second end and the first end.

8. An apparatus comprising a device according to any of claims 1 to 7, wherein the device is installed in a compatible vessel, preferably a laboratory tube.

9. A method for concentrating buoyant material, comprising the steps of:
a) immersing a sample of material in a liquid held within a cooperating vessel;
b) installing a device for concentrating buoyant biological material in the cooperating vessel, wherein the device comprises a body having a first end and a second end and walls therebetween defining an internal space,
wherein the first end has a first portal therein;
wherein the second end has a second portal therein;
wherein the port in the first end is in fluid connection with the space;
wherein the port in the second end is in fluid connection with the space;
wherein the fluid connection between the first and second portals is occluded by a filter or mesh mounted at the second end or between the first and second ends to extend over a transverse area of the space at the second end or between the first and second ends
wherein the cross sectional area of the space narrows between the second end and the first end
wherein the exterior dimensions of the of the second end of the device are for slidable installation or seating in a cooperating vessel; and
c) wherein the second end of the device is installed first in the cooperating vessel
d) urging the device towards the base of the cooperating vessel such that the filter or mesh is submerged in the liquid and the meniscus of the liquid is driven upwards into the region of the device between the second end and the first end where the cross sectional area of the space narrows.

10. A method according to claim 9, wherein driving the liquid into the region of the device where the cross sectional area of the space narrows reduces the area of the meniscus and thus concentrates the material floating at the air-liquid interface.

11. A method according to claim 9 or claim 10, wherein the liquid has a specific gravity of 1.18 to 1.26.

12. A method according to any of claims claim 9 to 11, wherein the sample is a biological sample, preferably a stool or faecal sample.

13. A method according to any of claims 9 to 12, wherein the method additionally comprises the step of collecting liquid or material from the upper surface of the liquid.

14. A method according to any of claims 9 to 12, wherein the method additionally comprises the step of testing the liquid or material for the presence or prevalence of helminths and/or eggs thereof, protozoa, protozoa and eggs/cysts, giardia, lung worm larvae, Cryptosporidium, tapeworm eggs, and or nematode eggs.

15. An injection moulding tool for manufacturing a device as described in any of claims 1 to 7.
